# EUROPEAN PATENT SPECIFICATION

(11) **EP 2 175 833 B1**
(45) Date of publication and mention of the grant of the patent: **25.06.2014**
(21) Application number: 08834594.7
(22) Date of filing: 12.08.2008
(51) Int. Cl.: A61K 8/97, A61Q 19/08, A61Q 19/00, A61K 8/73

(54) **DERMOCOSMETIC COMPOSITIONS BASED ON TAMARIND SEED POLYSACCHARIDE AND VEGETABLE EXTRACTS**
HAUTKOSMETISCHE ZUSAMMENSETZUNGEN BASIEREND AUF POLYSACCHARIDEN AUS TAMARINDENKERNEN UND PFLANZLICHEN EXTRAKTEN
COMPOSITIONS DERMOCOSMÉTIQUES À BASE DE POLYSACCHARIDE DE GRAINE DE TAMARIN ET D'EXTRAITS VÉGÉTAUX

(30) Priority: 13.08.2007 IT RM20070441
(43) Date of publication of application: 21.04.2010
(73) Proprietor: OFTAGEN SURGICAL S.r.l., 56121 Pisa (PI) (IT)
(72) Inventor: SANSO' Marco, 20132 Milano (IT); COCCHI Luciano, 22155 Milano (IT)
(74) Representative: Banchetti, Marina
(86) International application number: PCT/IT2008/000549
(87) International publication number: WO 2009/040847

(56) References cited:
- JP-A- 10 167 951
- US-A- 5 876 729
- US-A1- 2002 168 425
- US-B1- 6 197 318
- FEVRIER F: "FAGUS SILVATICA EXTRACT. \NATURE, INNOVATION, PERFORMANCE PURELY AND SIMPLY A CLOSE ENCOUNTER" SOFW-JOURNAL SEIFEN, OELE, FETTE, WACHSE, VERLAG FUR CHEMISCHE INDUSTRIE, AUGSBURG, DE, vol. 119, no. 3, 1 February 1993 (1993-02-01), page 142,144,145,147, XP000343313 ISSN: 0942-7694

## Description

The present invention concerns dermocosmetic compositions based on tamarind seed polysaccharide and vegetable extracts. More specifically, the invention relates to a combination of functional substances of vegetable origin, the first consisting of a polysaccharide, which is present in high concentration in the material of natural origin known as tamarind gum, and the second consisting of a beech tree buds extract, which contains a pool of medium-low molecular weight molecules, among which flavonoids, polyphenols, water-soluble peptide fractions, amino-acids and mineral salts. Said combination is useful for the manufacture of cosmetic products having moisturizing, conditioning, energizing and mineralizing action, and which present a high anti-wrinkle effectiveness.

As it is known, the trend of the cosmetic science has evolved in relatively recent times towards a privileged employment of functional substances of a vegetable origin, purified and having proven activity and safety, and which, in addition, may be obtained by means of production techniques that do not negatively affect the environment.

Among the plenty bio-polymers in the vegetable kingdom that may comply with such requirements, a considerable importance in the dermocosmetic field is held by polysaccharides. In particular, it is well known that several polysaccharides are used in cosmetic preparations in view of their wetting and conditioning properties, their ability to form protective films when applied on the skin and their resulting activity as moisturizing agents. Some polysaccharides are also particularly useful for their ability to form gels, taking up a remarkable amounts of water. The most known examples of polysaccharide products used in cosmetics include hyaluronic acid, chitin, cellulose, carrageenans, agar. All these substances are included in the cosmetic formulations with functions of softening, moisturizing, protective and film-forming agents.

A natural polysaccharide polymer that may be considered included in the cited category is the polysaccharide obtained from the seeds of the tamarind tree, the *Tamarindus indica,* an evergreen plant that may reach 15 m of height and that produces pod fruits, widespread in India, Africa and all the Far East, where is cultivated primarily for food production, for the productions of preserves, extracts, sauces (i.e. chutney) and confections starting from the fruit. The latter contains big seeds having a high percentage of polysaccharides, that have the function of accumulating and preserving vital and energetic substances.

The tamarind seed, which was considered originally a by-product, has found various applications, once ground to the powder form (known as "tamarind gum" or "tamarind kernel powder"). The most important of such applications are in textile industry and in paper industry, where tamarind gum is employed as sizing and sticking agent, and in food industry, where it is used as thickening, gelling, stabilising and binding agent in any kind of products, as do other polysaccharide products such as alginates, pectines, guar gum or locust bean gum. Raw tamarind kernel powder, which is commercially available as such, contains from 65 to 73% by weight of polysaccharide, from 15 to 23% of protein material, from 3 to 8% of fats and oil and from 2 to 4% of ashes, besides minor amounts of crude fibre, tannins and other impurities.

According to several studies carried out on the structure of the polysaccharide fraction of tamarind gum, it is ascertained that tamarind seed polysaccharide consists of a main chain of glucopyranosyl units bound to each other through (1→4) linkages, with short side chains consisting of xylopyranosyl units attached to the main chain through (1→6) linkages (xyloglucan). Said xylopyranosyl units may be single, or they may be bound, in turn, to single galactopyranosyl units through a (1→2) linkage. The further presence of arabinofuranosyl units has also been reported. Thus, the structure of the tamarind seed polysaccharide (also referred to in the following as TSP) may be represented as follows:

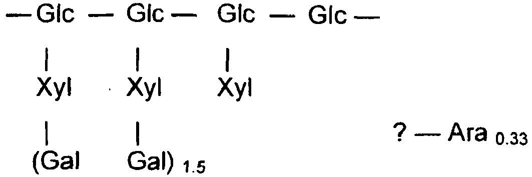

As it will be illustrated in greater detail further on, the procedure for the extraction, filtration and purification of the polymer from the starting raw material requires a high technological specialization in order to maintain unchanged the functional characteristics also in view of the high molecular weight of the polysaccharide, which in a correctly purified product ranges from 600,000 to 750,000 Da

An example of use of the *Tamarindus indica* polysaccharide in cosmetics is shown in the patent application JP-A-10167951 (Dainippon Pharmaceutical Co.), concerning a product suitable in cosmetic formulations for skin and hair care, containing from 0.005 to 10% by weight of the *Tamarindus indica* polysaccharide (partially hydrolyzed) and a saccharide and/or a polyhydroxy alcohol.

A further example in the same field is represented by the European patent EP 0720848 (Cognis France S.A.), which discloses the use of tamarind seed extracts enriched with xyloglycans as active substances in cosmetic or dermatologic preparations intended for application on the skin, with the function of skin softening, moisturizing and film-forming agents, able to give to the skin a smooth and velvety aspect, and also to show a long term activity as immunomodulator and immunostimulant substances. Corresponding products are marketed by the same holder with the trademark IMINDINYL^{®} (INCI name of the active substance: *Tamarindus Indica* Seed Polysaccharide).

Another cosmetic product based on TSP as fundamental ingredient is disclosed in the patent application EP 0965321 (Shiseido Company Ltd.), concerning a composition for external use including xyloglycan extracted from the tamarind seed as fundamental ingredient, together with a water-soluble UV filter. The described compositions also include, preferably, a polysaccharide thickening agent (selected, in particular, from hydroxyethylcellulose, xanthan gum, agar, tragacanth gum and locust bean powder) and further functional substances selected from sericine, carboxyvinyl polymers and alkylcarboxyvinyl polymers. In the disclosed compositions the combination of xyloglycan and UV filter affords an improved softness to the touch and reduces the sticky feeling that is typical of many compounds used as sunscreens.

Therefore, on the basis of the known technique, the main activity of TSP in the cosmetic field appears to be that of protecting the skin from the loss of trans-epidermal water and of assuring a regular hydration to the corneocytes. This is possible because of the high amounts of water that the polymer itself is able to take up and coordinate, and this ability is maintained when the polysaccharide is distributed on the skin through a cosmetic product. The natural skin defense mechanisms are also improved and the hydrolipidic barrier functionality is optimized, with an increase of the skin elasticity, softness and smothness.

In view of the foregoing, it is the object of the present invention to provide a dermocosmetic preparation based on functional substances of natural origin, specifically substances extracted from the vegetal kingdom, particularly suitable for skin application, having not only a conditioning, film-forming and moisturizing action, but also a tonic and revitalizing action, which preparation combines the action due to the polysaccharide macromolecule of TSP with a supplementary anti-wrinkle action and the ability to stimulate the vital functionalities of the skin.

To this aim, within the studies that lead to the present invention, there has been considered the possibility of combining the tamarind seed polysaccharide with a vegetal complex already used in cosmetics for its anti-wrinkle and skin-nutritive properties, i.e. the extract from the beech tree buds (*Fagus sylvatica*), in order to manufacture preparations for skin care which are particularly suitable for anti-age and anti-wrinkle treatment, specifically for the face.

The vegetal extract that is obtained from the beech tree buds, marketed under the trademark Gatuline^{®} RC from Gattefossé (INCI name of the active substance: Water (and) *Fagus sylvatica* Extract) is a vegetal complex rich of water-soluble peptide factors obtained from the buds of the beech tree, a plant that may reach the adult age in about sixty years, and that normally lives up to one hundred fifty-two hundreds years, reaching the average height of 5 m.

According to the procedure provided for manufacturing the extract, the buds are harvested in wild and semi-mountain areas, far from the urban and industrial zones, in order to avoid the effects of environment pollution, and also far from zones with grain, fruit trees and vegetable cultivations, in order to avoid the contamination due to the use of pesticides and herbicides in these cultivations. The harvest is performed during a very short period of gemmation (1-2 weeks), when the tissues are richest of vital energetic substances. The buds are harvested only from the lower branches in order not to interfere with the future growth of the tree.

The extraction process of the active substances from the buds thus harvested is described in the European patent EP 0470017 (Gattefossé S.A., corresponding to the US patent US 6270773, Pourrat et al.), having the title: Process for stabilizing vegetable plants. The fresh buds are frozen on the collection place to avoid degradation during the transfer to the production site where the extraction is performed, thus assuring that the composition of the final extract is the same of the fresh plant. Here, the buds are defrosted, rehydrated and treated with a stabilizing process based on the hyper frequencies (UHF, Ultra High Frequencies, electromagnetic waves with an average length of about 1 centimeter and with a frequency comprised between 300 MHz and 3 GHz; this range, in particular, includes the waves produced by a microwave oven), to reach a temperature at which the naturally present enzymes which normally cause the vegetable tissue deterioration are inhibited.

After the disclosed stabilizing treatment, the buds undergo several extraction treatment steps in order to yield the active fraction, rich in flavonoids, polyphenols, water-soluble peptide fractions, amino acids and mineral salts, and which contains, in particular, highly energetic active agents such as phytohormones and phytostimulines, that are responsible for stimulating the cell metabolism that activates the passage from the dormant stage to the bud development. Actually, Gatuline^{®} RC results from an updated revisitation of gemmotherapy, a therapeutic method based on the administration of fresh vegetable tissues at the embryonic stage, such as buds or young sprouts, the active principles of which are extracted from vegetal material through a suitable solvent. Such method originated from the finding that the embryonic vegetable tissues contain remarkable quantities of components such as auxines and gibberellins (phytohormones), or such as some enzymes and proteins, that are often present only in trace amounts in the adult parts of the plants.

Thus, it was ascertained that the beech tree buds extract, in the form marketed as Gatuline^{®} RC, when used in products for topical application on the skin, causes an improvement of cell metabolism, and has a re-epitelizing, anti-oxidant, revitalizing and stimulating action. In view of these properties, the said active substance is included as fundamental ingredient in "anti-age" cosmetic products, with anti-wrinkle activity.

In spite of the fact that the properties of the tamarind gum polysaccharide and of the beech tree buds extracts were already known, the possibility to combine together such two functional substances in a single dermocosmetic preparation had never been considered before. In addition, a property of synergic potentiation of the hydrating, revitalizing and anti-wrinkle activity of these two functional substances when combined together had not been evidenced before. It has now been found that a combination of TSP (*Tamarindus Indica* Seed Polysaccharide) with the beech tree buds extract (Water (and) *Fagus sylvatica* Extract), used in a product for skin care, results in a remarkable synergistic activity, which is likely to be due to the mutual complementarity of the two functional components of the preparation. Such synergistic activity may be proven experimentally by ascertaining the potentiated ability to improve the elastic properties of the skin, to increase the cutaneous hydration index, to reduce the cutaneous micro-roughness and to improve the skin texture.

Therefore, the present invention specifically provides a cosmetic composition comprising, as functional ingredients, from 0.1 to 2% by weight of tamarind seed polysaccharide (*Tamarindus Indica* Seed Polysaccharide) and from 0.5 to 15% by weight of beech tree buds extract (Water (and) *Fagus sylvatica* Extract). In such compositions, the highest levels of the beech tree buds extract, up to 15% by weight, are provided for specific hyper-concentrated products, but the most generally preferred compositions according to the invention are those which comprise from 0.1 % to 1% by weight of tamarind seed polysaccharide and from 0.5 to 5% by weight of beech tree buds extract.

Specifically, as it results from the foregoing, the cosmetic composition according to the invention contains the beech tree buds extract referred to as Gatuline^{®} RC. Said product has an indicative composition which comprises water soluble peptide components, flavonoids, polyphenols, mineral salts (in particular calcium, magnesium, potassium, silicon salts) and 0.45% by weight of Phenonip, the latter being a preservative consisting of an optimized admixture of paraben (methyl-, ethyl-, propyl-, butyl- and isobutyl-paraben) esters in phenoxyethanol.

A certified organic version of Gatuline^{®} RC, having the name Gatuline^{®} RC Bio is also available on the market. The latter is free from ethoxylated agents, and its preservative system is obtained by means of benzyl alcohol.

According to a preferred embodiment of the invention, the activity of which will be better disclosed herein in the following experimental section, the composition according to the present invention comprises 0.3% by weight of tamarind seed polysaccharide and 3% by weight of Gatuline^{®} RC.

In respect of a possible mechanism of action of the functional ingredients combination according to the invention, in terms of molecular weight, the tamarind seed polymer acts as a high molecular weight "carrier" for the pool of functional ingredients of the beech tree buds extract, which have a low molecular weight. This presumably allows to obtain, from the application of the combination, results that are better than those expected on the basis of the properties of the two ingredients individually taken.

Although it is not intended to be bound by a specific theory, it is hypothesized that the combination performs a combined action on two levels, i.e. a superficial action and a deeper one, on the basis of two distinguished mechanisms: the polysaccharide polymer covers the skin with a film coordinating high amounts of water and producing a protective and moisturizing action, while the beech tree extract ingredients complex, having a lower molecular weight, penetrates into the lower layers, promoting hydration and skin elasticity through cell renewal and natural protein synthesis. As a consequence, the skin treated with the combination appears remarkably more hydrated, refinished, elastic, compact and enlightened, confirming the hypothesis of a synergetic action of the multifunctional complex of the invention.

As already noted, by the term "tamarind seed polysaccharide" as used in the present application, it is meant a fraction enriched in polisaccharides obtainable from the tamarind gum (or tamarind kernel powder), the latter being the raw product normally available on the market, produced by grinding of *Tamarindus indica* seeds, according to technologies that had the first developments in India. A partially purified polysaccharide fraction of the tamarind gum is marketed, for example, by Dainippon Sumitomo Pharma Ltd. di Osaka, Japan, under the trademark Glyloid^{®}. For the purposes of the present invention, however, the polysaccharide fraction at issue is preferably further purified to give a practically pure tamarind seed polysaccharide.

A method for the production of purified tamarind seed polysaccharide suitable for the use in the cosmetic preparation according to the invention, starting from partially purified tamarind gum products available in the market (as for example, the cited Glyloid^{®}) consists in dispersing the starting product in cold deionized water, stirring for 12 hours in order to obtain a homogeneous dispersion. In order to separate by precipitation any possible proteins present, the dispersion thus obtained is heated to 80°C in 30 minutes and, after cooling, is centrifuged at 5000 r.p.m. for 30 minutes. The supernatant solution is then dialyzed against water for at least 48 hours at 4°C, using molecular weight cut-off membranes of 12,000-14,000 Da. The resulting solution is finally lyophilized in order to obtain a final white translucent product, completely water-soluble. The absence of contaminating proteins is ascertained by sodium dodecylsulfate on polyacrylamide gel electrophoresis (SDS-PAGE).

However, other purification processes are known in the art, specifically in connection with the use of tamarind gum or tamarind gum polysaccharide in other productive fields, in particular for the known pharmaceutical use of TSP which is the object of the European patent EP 0892636 (Farmigea S.p.A.) and the corresponding US patent 6056950. The polysaccharide may also be advantageously purified by any advanced separation or purification process suitable to eliminate traces of proteins or other contaminants, which may offer products of particularly high purity.

By the term "beech tree buds extract" it is meant, within the scopes of the present invention, the already described product of vegetal origin corresponding to the INCI denomination "Water (and) *Fagus sylvatica* Extract", and also, specifically, the preservative-additioned commercial products which correspond to the trademark Gatuline^{®} RC e Gatuline^{®} RC Bio. An operating procedure to afford the said products has been previously described.

The composition containing the two functional ingredients according to the invention may be presented in all the galenical forms normally used for a topical application on the skin, in particular, in the form of a aqueous solution, an oil-in-water or water-in-oil or a multiple emulsion, a silicone emulsion, a micro-emulsion, a nano-emulsion, or a aqueous gel.

The preparation, more or less fluid, which results therefrom may be formulated, in particular, as cream, milk, lotion, serum or gel. Said preparation may be, specifically, an anti-age cream for the face, a cream for the eye contour, a moisturizing cream for the face or the body, a sunscreen product, an after-sun cream or milk, a cream for the hands, a moisturizing aftershave product or anti-age or similar. As it results from the experiments shown below, the proposed composition is optimally suited to the manufacture of dermocosmetic preparations having anti-wrinkle and moisturizing activity.

According to a further aspect thereof, the invention provides the use of a combination of tamarind seed polysaccharide (*Tamarindus Indica* Seed Polysaccharide) and a beech tree buds extract (Water (and) *Fagus sylvatica* Extract) for the production of a dermocosmetic preparation having anti-wrinkle and moisturizing activity, and preferably a preparation that comprises from 0.1 % to 2% by weight of tamarind seed polysaccharide and from 0.5% to 15% by weight of beech tree buds extract.

As already noted, the beech tree buds extract is preferably represented by Gatuline^{®} RC. According to a specific embodiment of the invention, the preparation is an anti-wrinkle and moisturizing cream, and comprises 0.3% by weight of tamarind seed polysaccharide and 3% by weight of Gatuline^{®} RC.

The composition according to the invention may contain adjuvants customary in cosmetic and dermatologic technology, such as fatty substances, emulsifyers and co-emulsifyers, hydrophilic or lipophilic gelling agents, wetting agents and solvents, other secondary functional substances, preservatives, anti-oxidants, inert fillers, perfumes, dyes, neutralizing agents, permeation enhancers. The proposed amounts of these adjuvants are those normally used in the cosmetic and dermatologic technology, generally from 0.01% to 30% by weight on the overall weight of the composition.

The fatty substances to be used in the composition according to the invention may include vegetal oils (such as almond, jojoba and avocado oil), mineral oils, oils of animal origin (such as lanoline), synthetic oil (esters such as isopropyl myristate, decyl oleate, isopropyl palmitate), silicone oils (such as cyclomethicone and dimethicone) and fluorinated oils. Fatty alcohols and acids and waxes (such as bee wax and rice wax) may also be used.

The emulsifyers and co-emulsifyers to be used in the composition may include polyglycerol fatty acid esters, saccharose fatty acid esters, sorbitan fatty acid esters, ethoxylated sorbitan fatty acid esters, ethers of fatty alcohols and PEG, glycerol fatty acid ethers, alkyl sulfates, alkyl ether sulfates, alkyl phosphates, alkyl polyglucosides , dimethicone co-polyols. Examples of ethoxylated emulsifyers are steareth-2 and steareth-21, while examples of glucoside-based emulsifyers are cetearyl glucoside and arachidyl glucoside

The hydrophilic gelling agents normally employed are carboxy vinyl polymers (carbomers), acrylic copolymers such as acrylate/alkylacrilate copolymers, polyacrylamides, polysaccharides such as xanthan gum, carraggeenans, guar gum. The lipophilic agents to be used may include clays, fatty acid metal salts, hydrophobic silicon and ethyl cellulose.

The wetting agents and the solvents to be comprised in the formulation of the invention besides water may include, for example, glycerin, propylene glycol, butylene glycol, sorbitol, xylitol, betaine.

The supplementary functional substances which may be present are cheratolitic and/or desquamative agents, anti-acne agents, anti-seborroics, anti-irritants, drainage agents, slimming agents and vitamins and derivatives thereof (such as vitamin E or tocopherol, vitamin C, panthenol)

The preservative agents to be used in the composition according to the invention may comprise for example, benzoic acid and its salts and esters, sorbic acid and their salts, parabens and their salts, phenoxyethanol, triclosan or admixtures thereof. The antioxidant agents that may be used in the composition of the invention mainly include the chelanting agent EDTA and its salts.

The formulations according to the invention may also include inert fillers, for example talc, kaolin, magnesium carbonate or aluminum silicate, and UVA and UVB filters, including those generally used in the cosmetic and dermatologic technique, such as benzophenone-3, octyl methoxycinnamate, trisiloxane drometizole.

Dyeing agents, perfumes, and neutralizing substances to be used in the proposed combination are those which are customary in cosmetic and dermatologic technology. In particular, among the neutralizing agents, sodium hydroxide, triethanol amine, aminomethyl propanol, potassium hydroxide have to be cited.

Finally, as permeation enhancing agents, alcohols or glycols (such as ethanol and propylene glycol), fatty acids (such as oleic acid) and esters thereof (such as dimetyl-isosorbide) may be added to the formulation.

The specific features of the invention, as well as the advantages thereof, will become more apparent with reference to the detailed description presented below by way of example only, together with the results of the experimentations carried out thereon and with data for comparison with the prior art. Some experimental results are also shown in the accompanying drawings, wherein:
**Figure 1** shows, in histogram form, the results of an instrumental test for the evaluation of the skin elasticity obtained by applying a composition according to the invention, in comparison with each one of the two functional ingredients which are included therein, when individually applied;
**Figure 2** shows, in histogram form, the results of an instrumental test for the evaluation of the skin hydration obtained by applying a composition according to the invention, in comparison with each one of the two functional ingredients which are included therein, when individually applied; and
**Figures 3, 4****,** **5, 6** and **7** respectively show, in histogram form, the results of clinical tests for the evaluation of the following parameters: cutaneous micro-roughness, skin refinishing, flesh color; skin softness; cutaneous compactness for a composition according to the invention in comparison with each one of the two functional ingredients which are included therein.

### EXAMPLE 1

### Eye contour gel cream

An experimental formulation of the product according to the invention has the following composition:

| **INGREDIENTS (commercial name)** | **INCI name** | **Supplier** | **% w/w** |
|---|---|---|---|
| Osmosis water | Aqua | Sigmar | 75.030 |
| Disodium EDTA | Disodium EDTA | Vama | 0.050 |
| Glycerin | Glycerin | Vama | 2.000 |
| Panthenol | Panthenol | Res Pharma | 0.500 |
| Xanthan gum T | Xanthan gum | Faravelli | 0.250 |
| TSP (tamarind seed poly-saccharide) | *Tamarindus Indica* Seed Polysaccharide | Farmigea | 0.250 |
| NaOH 30% (soda) | Sodium hydroxide | Brenntag | 0.150 |
| Montanov 202 | Arachidyl alcohol, Behenyl alcohol, Arachidyl glucoside | Seppic | 5.000 |
| Lanol 99 | Isononyl isononanoate | Seppic | 6.000 |
| Almond oil | Prunus dulcis | Huwell | 2.000 |
| Lipex 106 | Shorea stenoptera | Huwell | 1.000 |
| Lanol P | Cetyl palmitate | Seppic | 1.000 |
| Aperoxid TLA | Lecithin, Tocopherol, Ascorbyl palmitate, Citric acid | Biochim | 0.020 |
| Sepilift DPHP | Dipalmitoyl hydroxyproline | Seppic | 0.700 |
| Dermosoft octiol | Caprylyl glycol | PCP | 1.000 |
| Vitamin E acetate | Tocopheryl acetate | Res Pharma | 0.500 |
| Sepicide HB2 | Phenoxyethanol, Methylparaben, Ethyl-paraben, Propylparben, Butylparaben, Isobutylparaben | Seppic | 0.500 |
| Gatuline RC | Fagus sylvatica extract | Gattefossé | 3.000 |
| Simulgel NS | Hydroxyethyl acrylate/Sodium acryloyldimethyl taurate | Seppic | 1.000 |
| | | | **100.000** |

The composition has a pH = 6.8-7.2 and appears to be a transparent aqueous solution, with a characteristic very light smell.

The procedure for the preparation of the cream is as follows.

In a turbo-emulsifyer dose the first 7 components of the list (Phase A), mixing with turbo and blades under vacuum until the dispersion of the polymers (xanthan gum and TS-P) is completed. Once the dispersion is completed, heat the mass, stirring with speedy blades until the temperature reaches 70-75°C.

Add the components of the Phase B (from the eighth to the third from the last component of the list) into a melter of suitable capacity, heating under gentle stirring to reach 70-75°C. Emulsify by slowly sucking the Phase B in the turbo-emulsifyer under vacuum and stirring with turbo and speedy blades. When the mixing of Phases A and B is completed, continue stirring with turbo and speedy blades under vacuum for 10-15 minutes, then begin the cooling stirring with the speedy blades only under vacuum. Continue until T < 40°C is reached.

When the temperature drops below 40°C, add the last two components of the list (Phase C) to the preparation, sucking them upwards under vacuum and stirring with turbo and speedy blades for 5-10 minutes. At the same time, continue cooling the mass, carrying on the stirring with slow blades, until T<28°C is reached. Pour the admixture into sanitized containers and wait for the separation in two phases.

### EXAMPLE 2

### Face serum

A formulation according to the invention is experimentally produced, having the following composition:

| **INGREDIENTS (commercial name)** | **INCI name** | **Supplier** | **% w/w** |
|---|---|---|---|
| Osmosis water | Aqua | Sigmar | 89.800 |
| Disodium EDTA | Disodium EDTA | Vama | 0.100 |
| Glycerin | Glycerin | Vama | 5.000 |
| Gatuline RC | Fagus sylvatica extract | Gattefossé | 0.500 |
| TSP (tamarind seed polysaccharide) | *Tamarindus Indica* Seed Polysaccharide | Farmigea | 1.000 |
| natrosol 250 HHR | Hydroxyethylcellulose | Eing & Ver | 0.800 |
| labrafac CC | Caprylic/Capric Triglyceride | Gattefossé | 2.000 |
| solrpogress70 | PEG-40 Hydrogenated castor oil | Prodotti Gianni | 0.200 |
| optiphen | phenoxyethanol, caprylyl glycol | Vama | 0.600 |
| | | | **100.000** |

### EXAMPLE 3

### Anti-age cream

A formulation suitable for an anti-wrinkle cream had the following composition:

| **INGREDIENTS (trade name)** | **% w/w** |
|---|---|
| Osmosis water | 70.750 |
| Montanov 202 | 5.000 |
| Lanol 99 | 10.000 |
| Sepilift DPHP | 1.000 |
| Monoi butter | 2.000 |
| Sepifeel One | 1.000 |
| Aquaxyl | 3.000 |
| Panthenol | 1.000 |
| TSP (tamarind seed polysaccharide) | 0.350 |
| Gatuline RC | 3.000 |
| Simulgel NS | 1.400 |
| Germall 115 | 0.250 |
| Sepicide HB | 0.300 |
| Perfume Pomme Miel | 0.250 |
| Tocopheryl Acetate | 0.600 |
| NaOH (sol. 30%) | 0.100 |
| | 100.000 |

### EXAMPLE 4

### Moisturizing cream for body

A formulation suitable for a moisturizing cram for body according to the invention had the following composition:

| **INGREDIENTS (trade name)** | % **w/w** |
|---|---|
| Osmosis water | 79.750 |
| Montanov L | 3.000 |
| Lanol 99 | 7.000 |
| Monoi butter | 1.500 |
| Lanol P | 0.500 |
| Aquaxyl | 3.000 |
| Panthenol | 0.500 |
| TSP (tamarind seed polysaccharide) | 0.150 |
| Gatuline RC | 2.000 |
| Simulgel NS | 0.800 |
| Germall 115 | 0.250 |
| Vit PP | 0.100 |
| Sepicide HB | 0.300 |
| Perfume mint tea | 0.500 |
| Tocopheryl Acetate | 0.600 |
| Menthyl lactate | 0.050 |
| | 100.000 |

### Clinical and instrumental test - Evaluation of the anti-wrinkle effectiveness

The study carried out allowed to evaluate the anti-wrinkle effectiveness of a cosmetic treatment that is to be used on the face skin. The products under study to be compared to each other are the following:
- Gel TS-P 0,3% (also referred to as T)
- Gel 3% Gatuline RC (also referred to as G)
- Gel TS-P 0,3% + 3% Gatuline RC (also referred to as T+G)

The following cutaneous parameters were measured in the subjects of the test groups:
- Skin elasticity
- Skin hydration index
- Cutaneous profilometry

The instrumental analysis was completed with the clinical examination by a dermatologist, with the evaluation of the following parameters:
- Clinical analysis: .
   1. Cutaneous micro-roughness
   2. Skin refinishing
   3. Flesh color
   4. Cutaneous compactness
   5. Skin softness
- Self-valuation
   1. Pleasantness
   2. Effectiveness
   3. Tolerability

The overall test group included 45 female subjects, aged in the range of from 30 to 65 years, showing signs of cutaneous micro-roughness and roughness. After the recruitment, the group was divided in three subgroups, each comprising 15 volunteers.

The test was performed by means of the method of the controlled use test, with the application of the product at home, with a frequency of one application per day. The clinical and instrumental measurements are collected before the first treatment (T0) and after 15 (T15), 30 (T30) e 60 (T60) days of use of the product.

### Instrumental examination of the skin elasticity

The skin elasticity was measured by means of a Cutometer^{®} SEM 575 (Courage + Khazaka Electronic GmbH). The measurement principle is based on the suction and elongation of the skin. The instrument generates a negative pressure which may vary between 250 e 500 mbar, and the cutaneous surface to be measured is sucked inside the probe hole by the negative pressure generated by the instrument. The deepness of penetration of the skin in the probe hole is determined, without contact, by means of an optical measurement system connected to a computer.

The skin is sucked in the probe hole during the measurement by a negative constant pressure (A). Then, the negative pressure is brought to 0 mbar and the skin returns to its original position (B). The curve obtained evidences the viscoelastic qualities of the skin, being the curve composed by a first part due to the elastic component and by a second part due to the viscoelastic and plastic component. Said two components are equivalent in young and elastic skins, while in the elderly skins the viscoelastic and plastic component becomes predominant.

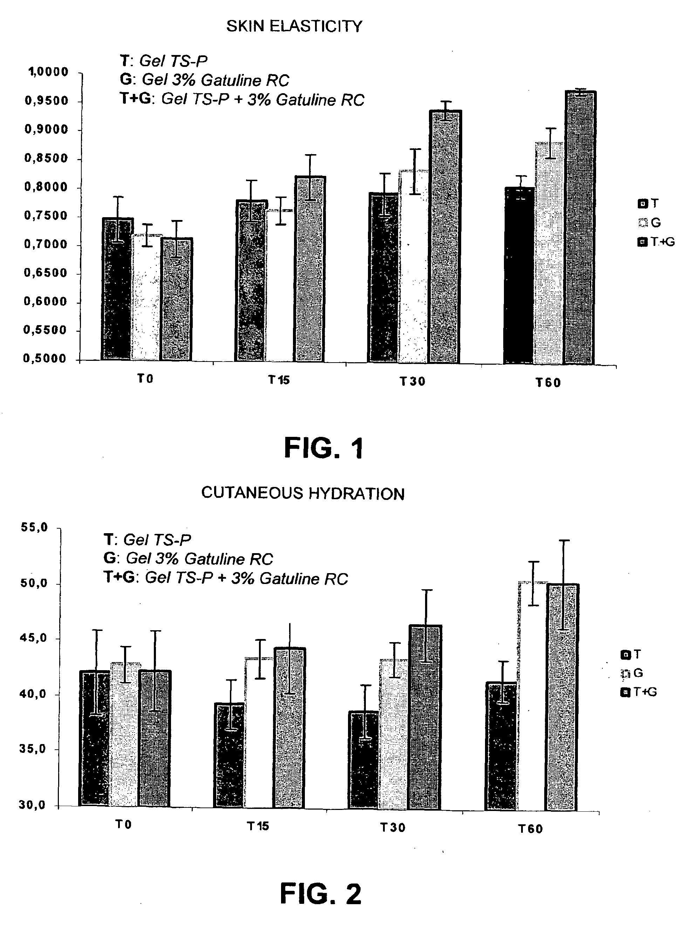

The skin elasticity is calculated in percent terms by the measurements performed as explained, and tends to 100% when the skin is mostly elastic. The results obtained for the three volunteers groups, which have tested the three preparations to be compared, are shown in a numeric form in the following table and in diagram form in the attached **Figure 1****.**

**TABLE 1**

| Measurement of skin elasticity | | | | | | | | | | | | |
|---|---|---|---|---|---|---|---|---|---|---|---|---|
| | **T0** | | | **T15** | | | **T30** | | | **T60** | | |
| | **T** | **G** | **T+G** | **T** | **G** | **T+G** | **T** | **G** | **T+G** | **T** | **G** | **T+G** |
| **Average** | 0.7472 | 0.7192 | 0.7135 | 0.7815 | 0.7636 | 0.8227 | 0.7941 | 0.8346 | 0.9397 | 0.8070 | 0.8858 | 0.9753 |
| **SEM** | 0.0394 | 0.0188 | 0.0318 | 0.0363 | 0.0237 | 0.0392 | 0.0359 | 0.0387 | 0.0169 | 0.0215 | 0.0254 | 0.0052 |
| **% vs T0** | | | | 4.49 | 6.18 | 15.31 | 6.27 | 11.70 | 25.76 | 8.00 | 18.54 | 30.51 |

From the above data, it is possible to ascertain that the values obtained at T60 differ in a statistically significant manner, as follows:
- The 3% Gatuline RC Gel determines an increase of skin elasticity that is higher than the one obtained with the Gel TS-P.
- The elasticity increase obtained with the TS-P 0,3% + 3% Gatuline RC Gel is higher than the values obtained with the gel TS-P and with the gel containing Gatuline RC only.

From the values obtained it is deduced that the two functional ingredients act in a synergetic way in increasing the skin elasticity.

### Instrumental examination of the skin hydration index

The measurement is based on the internationally recognized method Corneometer^{®}. The measurement is based on the different dielectric constant of water compared to other substances. The measurement probe shows the changes of the capacitance according to the water content of the object to be measured.

The results obtained for the three volunteers groups, which have tested the three preparations to be compared, are shown in a numeric form in the following table and in diagram form in the attached Figure 2.

**TABLE 2**

| Measurement of the skin hydration | | | | | | | | | | | | |
|---|---|---|---|---|---|---|---|---|---|---|---|---|
| | **T0** | | | **T15** | | | **T30** | | | **T60** | | |
| | **T** | **G** | **T+G** | **T** | **G** | **T+G** | **T** | **G** | **T+G** | **T** | **G** | **T+G** |
| **Average** | 42.1 | 42.8 | 42.3 | 39.3 | 43.4 | 44.4 | 38.8 | 43.5 | 46.6 | 41.6 | 50.5 | 50.4 |
| **SEM** | 3.8 | 1.6 | 3.6 | 2.2 | 1.8 | 4.0 | 2.4 | 1.5 | 3.2 | 1.9 | 2.0 | 4.0 |
| **% vs T0** | | | | -6.6 | 1.4 | 5.2 | -7.9 | 3.3 | 10.7 | -1.2 | 19.9 | 19.7 |

From the above data, it is possible to ascertain that the values obtained at T60 differ in a statistically significant manner, as follows:
- Both the gel TS-P 0,3% + 3% Gatuline RC and the gel with 3% Gatuline RC only produced a higher increase than that obtained by Gel TS-P, which when used alone produces a real reduction of the hydration.
- The increase of hydration obtained from the combination gel TS-P 0,3% + 3% Gatuline RC is higher than expected, in consideration of the fact that the contribute of TS-P alone was negative.

Also these data show a synergetic interaction of the two functional ingredients.

### Cutaneous profilometry

The reduction of the depth of the wrinkles has been evaluated instrumentally by means of a videocamera Visioscan VC 98 for the *in vivo* analysis of the skin surface. The parameters considered in the image analysis are: 1) the cutaneous coarseness or ruggedness, 2) the cutaneous squamousness, 3) the skin refinishing, 4) cutaneous roughness, which is proportional to the number and to the width of the wrinkles, and 5) the wrinkle volume, which is proportional to its depth.

From the instrumental values obtained for these five parameters for each one of the three products to compare (**T, G e T+G**) it resulted that the major variations in the cutaneous profilometric parameters are found in the test group which utilized the Gel TSP + 3% Gatuline RC.

### Clinical analysis

The clinical valuations have been collected by a dermatologist according to the scores shown in the following Tables.

| Cutaneous micro-roughness | |
|---|---|
| The micro-wrinkle are not visible | 1 |
| Light micro-roughness ascertainable with a video-camera with high magnification | 2 |
| Evident micro-roughness (also visible with naked eye) | 3 |

| Skin refinishing | |
|---|---|
| Not refinished skin | 1 |
| Poorly refinished skin | 2 |
| Refinished skin | 3 |
| Well refinished skin | 4 |

| Flesh-color | |
|---|---|
| Very dull color | 1 |
| Opaque skin | 2 |
| Tendentially opaque skin | 3 |
| Enlightened skin | 4 |

| Skin softness | |
|---|---|
| Rough skin | 1 |
| Tendentially chapped skin | 2 |
| Fairly soft skin | 3 |
| Soft skin | 4 |

| Cutaneous compactness | |
|---|---|
| Not compact (very low elasticity) | 1 |
| Insufficiently compact (low elasticity) | 2 |
| Compact (skin elasticity) | 3 |
| Well compact (good elasticity) | 4 |

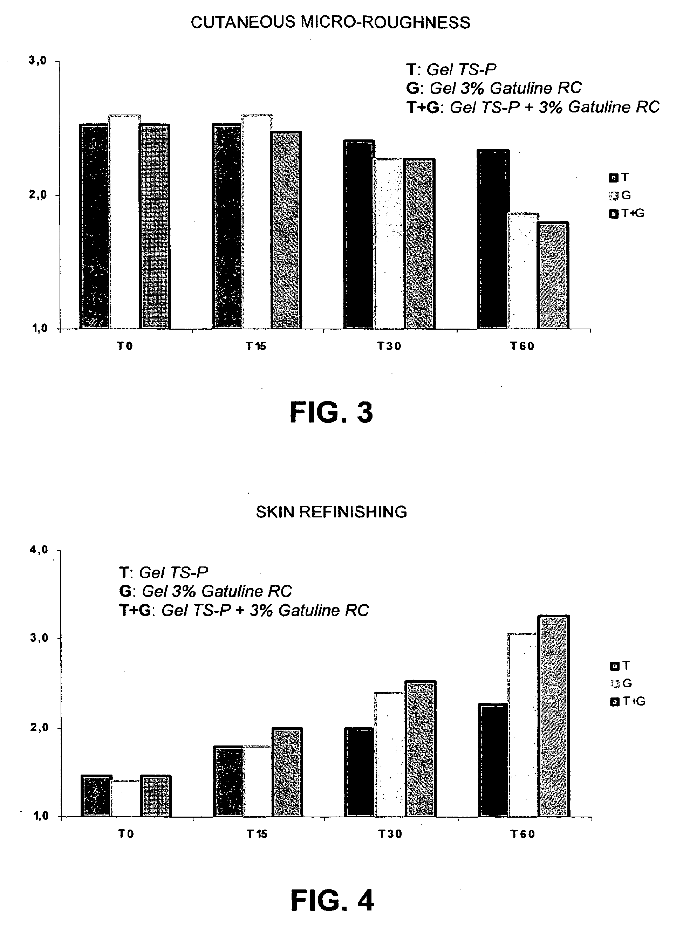

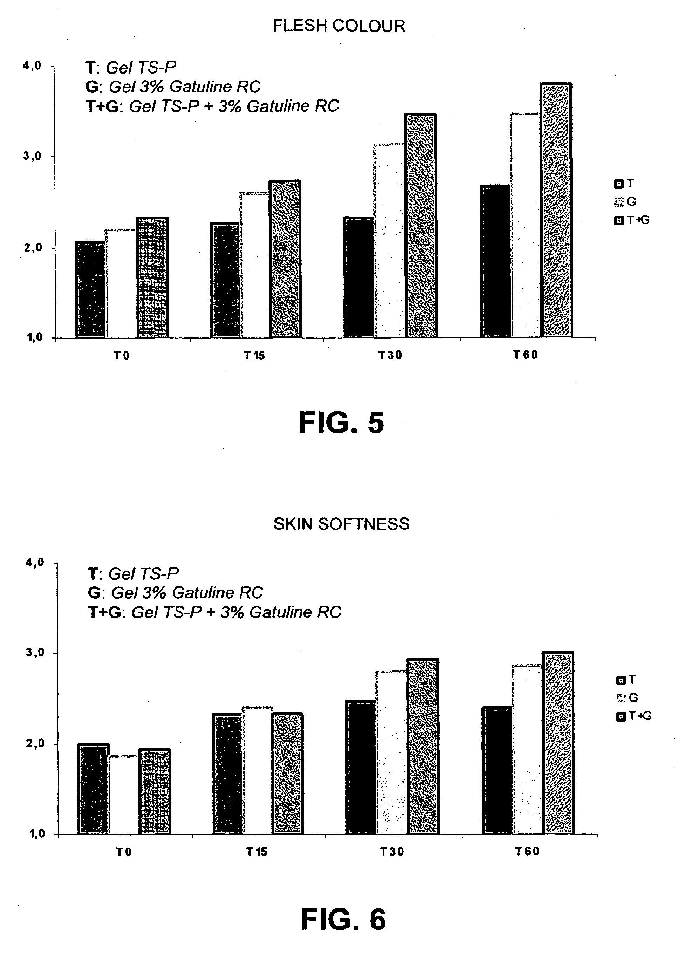

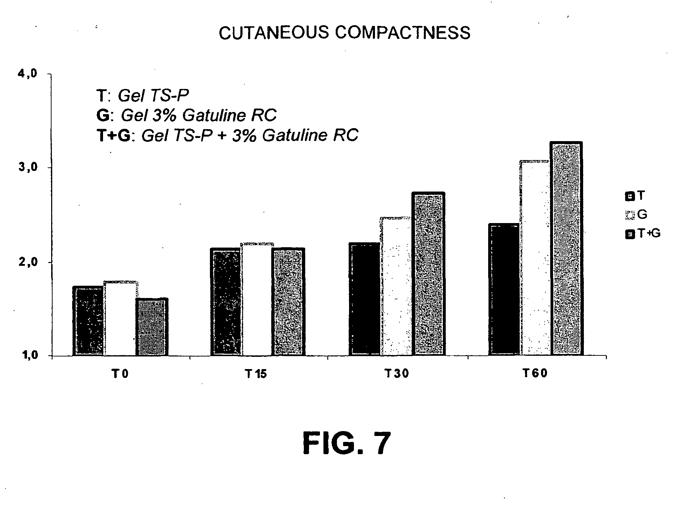

The average of the scores assigned on the basis of the clinical examination are shown, for each one of the three products to be compared (T, G e T+G) and for each one of the five parameters evaluated, in the attached **Figures 3, 4****,** **5, 6** and **7****,** while the following Tables 3-7 show the percent variation of the collected scores for each one of the parameters under study.

**TABLE 3**

| Percent variations - Cutaneous Micro-roughness | | | |
|---|---|---|---|
| | **T** | **G** | **T+G** |
| **T15** | 0.00 | -5.30 | -7.90 |
| **T30** | 0.00 | -12.80 | -28.20 |
| **T60** | -2.60 | -10.50 | -28.90 |

**TABLE 4**

| Percent variations - Skin refinishing | | | |
|---|---|---|---|
| | **T** | **G** | **T+G** |
| **T15** | 22.70 | 36.40 | 54.50 |
| **T30** | 28.60 | 71.40 | 119.00 |
| **T60** | 36.40 | 72.70 | 122.70 |

**TABLE 5**

| Percent variations - Flesh-color | | | |
|---|---|---|---|
| | **T** | **G** | **T+G** |
| **T15** | 9.70 | 12.90 | 29.00 |
| **T30** | 18.20 | 42.40 | 57.60 |
| **T60** | 17.10 | 48.60 | 62.90 |

**TABLE 6**

| Percent variations - Cutaneous softness | | | |
|---|---|---|---|
| | **T** | **G** | **T+G** |
| **T15** | 16.70 | 23.30 | 20.00 |
| **T30** | 28,600 | 50.00 | 53.60 |
| **T60** | 20.70 | 51.70 | 55.20 |

**TABLE 7**

| Percent variations - Cutaneous compactness | | | |
|---|---|---|---|
| | **T** | **G** | **T+G** |
| **T15** | 23.10 | 26.90 | 38.50 |
| **T30** | 22.20 | 37.00 | 70.8 |
| **T60** | 33.30 | 70.80 | 104.20 |

As it may be noticed both from the histograms of Figures 3-7 and from the previous percent values, the bigger variations in the detected clinical parameters are found in the group which used the product Gel TSP + 3% Gatuline RC.

### Self-valuation

At the end of the treatment the volunteers were asked to express a vote from 1 to 10 on Effectiveness, Pleasantness and Tolerability of the treatment. The available clinical scores for the volunteers to assign a personal judgment to the parameters at issue is a scale of values from 1 to 10 (1 means a very bad judgment and 10 means an optimum judgment, 6 means sufficient, and from 7 to 10 the judgments may be considered satisfactory).

The results obtained are synthesized in the following Table 8.

**TABLE 8**

| Self-valuation results | | | |
|---|---|---|---|
| | **Effectiveness** | **Pleasantness** | **Tolerability** |
| **T+P** | 8.3 | 8.4 | 9.1 |
| **T** | 6.0 | 7.5 | 8.7 |
| **G** | 8.0 | 8.1 | 8.9 |

As it may be noticed from the data synthesized above, from the performed experiment it is resulted that the cosmetic product Gel TSP 0.3% + 3% Gatuline RC demonstrated a higher effectiveness both in comparison with the cosmetic product Gel TSP and in comparison with the cosmetic product Gel 3% Gatuline RC in all the evaluated parameters. The obtained improvement levels in some of the parameters evaluated are in agreement with a synergetic effect of the two products when combined together, in suitable proportions, in a dermocosmetic product.

In particular, among the volunteers who underwent the clinical tests, the cosmetic product Gel TSP 0.3% + 3% Gatuline RC showed a remarkably increased effectiveness in improving the elastic properties of the skin, in increasing the hydration index, in reducing the skin micro-roughness and improving the skin texture. The skin appears, therefore, more hydrated, refinished, elastic, compact and enlightened.

The present invention has been disclosed with particular reference to some specific embodiments thereof, but it should be understood that modifications and changes may be made by the persons skilled in the art without departing from the scope of the invention as defined in the appended claims.

## Claims

1. A cosmetic composition comprising, as functional ingredients, from 0.1 to 2% by weight of tamarind seed polysaccharide (*Tamarindus Indica* Seed Polysaccharide) and from 0.5 to 15% by weight of beech tree buds extract (Water (and) *Fagus sylvatica* Extract).

2. A cosmetic composition according to claim 1, comprising from 0.1 to 1% by weight of tamarind seed polysaccharide and from 0.5 to 5% by weight of the beech tree buds extract.

3. A cosmetic composition according to claims 1 or 2, wherein the said beech tree buds extract is represented by Gatuline^{®} RC.

4. A cosmetic composition according to claim 3, comprising 0.3% in by weight of tamarind seed polysaccharide and 3% by weight of Gatuline^{®} RC.

5. A cosmetic composition according to any one of claims 1-4, presented in a cosmetic preparation in the form of a cream, milk, lotion, serum or gel.

6. A cosmetic composition according to any one of claims 1-5, consisting in an aqueous solution, an emulsion oil-in-water, an emulsion water-in-oil or a multiple emulsion, a silicone emulsion, a micro-emulsion, a nano-emulsion, or an aqueous gel.

7. A cosmetic composition according to any one of claims 1-6, presented in a dermocosmetic preparation having anti-wrinkle and moisturizing activity.

8. Use of a combination of tamarind seed polysaccharide (*Tamarindus Indica* Seed Polysaccharide) and a beech tree buds extract (Water (and) *Fagus sylvatica* Extract) for the manufacture of a dermocosmetic preparation having anti-wrinkle and moisturizing activity.

9. Use according to claim 8, wherein said preparation comprises the following functional ingredients in the following proportions: from 0.1 to 2% by weight of tamarind seed polysaccharide and from 0.5 to 15% by weight of beech tree buds extract.

10. Use according to claims 8 or 9, wherein said beech tree buds extract is represented by Gatuline^{®} RC.

11. Use according to claim 10, wherein said preparation comprises 0.3% by weight of tamarind seed polysaccharide and 3% by weight of Gatuline^{®} RC.

## Patentansprüche

1. Eine kosmetische Zusammensetzung, die als funktionelle Inhaltsstoffe umfasst: von 0,1 bis 2 Gew.-% Tamarindensamen Polysaccharid (*Tamarindus Indica* Samen Polysaccharid) und von 0,5 bis 15 Gew.-% Buchenknospenextrakt (Wasser (und) *Fagus sylvatica-Extrakt*).

2. Eine kosmetische Zusammensetzung nach Anspruch 1, umfassend: von 0,1 bis 1 Gew.-% Tamarindensamen Polysaccharid und von 0,5 bis 5 Gew.-% Buchenknospenextrakt.

3. Eine kosmetische Zusammensetzung nach Anspruch 1 oder 2, wobei der Buchenknospenextrakt durch Gatuline^{®} RC dargestellt wird.

4. Eine kosmetische Zusammensetzung nach Anspruch 3, umfassend 0,3 Gew.-% Tamarindensamen Polysaccharid und 3 Gew.-% Gatuline^{®} RC.

5. Eine kosmetische Zusammensetzung nach einem der Ansprüche 1 bis 4, dargeboten in einer kosmetischen Zubereitung in Form einer Creme, Milch, Lotion, Serum oder Gel.

6. Eine kosmetische Zusammensetzung nach einem der Ansprüche 1 bis 5, bestehend aus einer wässrigen Lösung, einer Öl-in-Wasser-Emulsion, einer Wasser-in-Öl-Emulsion oder einer multiplen Emulsion, einer Silikon-Emulsion, einer Mikroemulsion, einer Nanoemulsion, oder eines wässrigen Gels.

7. Eine kosmetische Zusammensetzung nach einem der Ansprüche 1 bis 6, dargeboten in einer hautkosmetischen Zubereitung, aufweisend eine Anti-Falten- und Feuchtigkeitsaktivität.

8. Verwendung einer Kombination von Tamarindensamen Polysaccharid (*Tamarindus Indica* Samen Polysaccharid) und einem Buchenknospenextrakt (Wasser (und) *Fagus sylvatica-Extrakt)* für die Herstellung einer hautkosmetischen Zubereitung, aufweisend eine Anti-Falten und Feuchtigkeitsaktivität.

9. Verwendung nach Anspruch 8, wobei die Zubereitung die folgenden funktionalen Inhaltsstoffe in den folgenden Anteilen enthält: von 0,1 bis 2 Gew.-% Tamarindensamen Polysaccharid und von 0,5 bis 15 Gew.-% Buchenknospenextrakt.

10. Verwendung nach Anspruch 8 oder 9, wobei der Buchenknospenextrakt durch Gatuline^{®} RC dargestellt ist.

11. Verwendung nach Anspruch 10, wobei die Zubereitung 0,3 Gew.-% von Tamarindensamen Polysaccharid und 3 Gew.-% von Gatuline^{®} RC umfasst.

## Revendications

1. Composition cosmétique comprenant, en tant qu'ingrédients fonctionnels, de 0,1 à 2% en poids de polysaccharide de graines de tamarin (Polysaccharide de Graines de *Tamarindus Indica*) et de 0,5 à 15% en poids d'extrait de bourgeons de hêtre (Eau (et) Extrait de *Fagus sylvatica*).

2. Composition cosmétique selon la revendication 1, comprenant de 0,1 à 1% en poids de polysaccharide de graines de tamarin et de 0,5 à 5% en poids d'extrait de bourgeons de hêtre.

3. Composition cosmétique selon la revendication 1 ou la revendication 2, dans laquelle ledit extrait de bourgeons de hêtre est représenté par la Gatuline^{®} RC.

4. Composition cosmétique selon la revendication 3, comprenant 0,3% en poids de polysaccharide de graines de tamarin et 3% en poids de Gatuline^{®} RC.

5. Composition cosmétique selon l'une quelconque des revendications 1-4, présentée dans une préparation cosmétique sous forme de crème, de lait, de lotion, de sérum ou de gel.

6. Composition cosmétique selon l'une quelconque des revendications 1-5, constituée par une solution aqueuse, une émulsion huile dans l'eau, une émulsion eau dans l'huile ou une émulsion multiple, une émulsion de silicone, une microémulsion, une nanoémulsion, ou un gel aqueux.

7. Composition cosmétique selon l'une quelconque des revendications 1-6, présentée dans une préparation dermocosmétique ayant une activité antirides et hydratante.

8. Utilisation d'une combinaison de polysaccharide de graines de tamarin (Polysaccharide de Graines de *Tamarindus Indica*) et d'un extrait de bourgeons de hêtre (Eau (et) Extrait de *Fagus sylvatica*) pour la fabrication d'une préparation dermocosmétique ayant une activité antirides et hydratante.

9. Utilisation selon la revendication 8, où ladite préparation comprend les ingrédients fonctionnels suivants dans les proportions suivantes: de 0,1 à 2% en poids de polysaccharide de graines de tamarin et de 0,5 à 15% en poids d'extrait de bourgeons de hêtre.

10. Utilisation selon la revendication 8 ou la revendication 9, où ledit extrait de bourgeons de hêtre est représenté par la Gatuline^{®} RC.

11. Utilisation selon la revendication 10, où ladite préparation comprend 0,3% en poids de polysaccharide de graines de tamarin et 3% en poids de Gatuline^{®} RC.
